(19) **Europäisches Patentamt / European Patent Office / Office européen des brevets**

(11) **EP 1 430 322 B1**

(12) **EUROPEAN PATENT SPECIFICATION**

(45) Date of publication and mention
of the grant of the patent:
**24.06.2009 Bulletin 2009/26**

(51) Int Cl.:
*G01R 33/28* [(2006.01)]    *A61B 5/055* [(2006.01)]

(21) Application number: **02766248.5**

(22) Date of filing: **06.09.2002**

(86) International application number:
**PCT/US2002/028431**

(87) International publication number:
**WO 2003/024324 (27.03.2003 Gazette 2003/13)**

(54) **Use of polarised 129XE in the manufacture of a medicament for use in an in-vivo NMR method**

Verwendung von polarisiertem 129Xe zur Herstellung eines Medikaments zur Verwendung in einem in-vivo-NMR-Verfahren

Utilisation de 129Xe pour la fabrication d'un médicament pour l'utilisation dans un procédé in vivo RMN

(84) Designated Contracting States:
**AT BE BG CH CY CZ DE DK EE ES FI FR GB GR IE IT LI LU MC NL PT SE SK TR**

(30) Priority: **20.09.2001 US 323667 P**

(43) Date of publication of application:
**23.06.2004 Bulletin 2004/26**

(73) Proprietor: **Medi-Physics, Inc.**
**Princeton, NJ 08540 (US)**

(72) Inventors:
• **DRIEHUYS, Bastiaan**
**Chaper Hill, NC 27516 (US)**
• **HALL, Margaret**
**L. Kingshill,**
**Buckinghamshire HP16 0EB (GB)**
• **MARELLI, Claudio**
**Buckinghamshire HP6 5AS (GB)**

(74) Representative: **Canning, Lewis R. et al**
**GE Healthcare Limited**
**Amersham Place**
**Little Chalfont,**
**Bucks. HP7 9NA (GB)**

(56) References cited:
**US-A1- 2001 000 727**

• **SWANSON S D ET AL: "DISTRIBUTION AND DYNAMICS OF LASER-POLARIZED 129XE MAGNETIZATION INVIVO" MAGNETIC RESONANCE IN MEDICINE, ACADEMIC PRESS, DULUTH, MN, US, vol. 42, no. 6, December 1999 (1999-12), pages 1137-1145, XP000870811 ISSN: 0740-3194**
• **WAGSHUL M E ET AL: "IN VIVO MR IMAGING AND SPECTROSCOPY USING HYPERPOLARIZED" MAGNETIC RESONANCE IN MEDICINE, ACADEMIC PRESS, DULUTH, MN, US, vol. 36, no. 2, 1 August 1996 (1996-08-01), pages 183-191, XP002070307 ISSN: 0740-3194**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**EP 1 430 322 B1**

**Description**

Field of the Invention

The present invention relates to magnetic resonance imaging ("MRI") and MR spectroscopy using hyperpolarized noble gases- More particularly, the present invention relates to techniques to assess certain conditions *in vivo* using polarized noble gases.

Background of the Invention

Chronic heart failure (CHR) appears to affect a relatively large and potentially increasing segment of the population. *See, e.g.,* The Task Force on Heart Failure of the European Society of cardiology. Guidelines for the Diagnosis of Heart Failure, 16 Eur. Heart Jnl., p. 741-751 (1995). In addition, many elderly heart failure patients are women, and the more common cause of the syndrome may be diastolic dysfunction. Early diagnosis of heart failure, particularly heart failure due to diastolic dysfunction, in which ejection fraction may be normal, remains a challenge. Documentation of pulmonary congestion in the absence of evidence for systolic dysfunction is believed to represent reasonable criteria for this diagnosis. *See* Tan et al., Heart Failure in Elderly Patients: Focus on diastolic Dysfunction, Heart Failure: Scientific Principles and Clinical Practice, P.A. (Churchill-Livingston, NY, Poole-Wilson, Ed. 1997). For other cardiopulmonary or respiratory disorders or diseases, patients may exhibit a shortness of breath of uncertain etiology that can make it difficult to identify the disorder or condition and/or to thus treat in an effective manner.

Early studies concerning imaging of dissolved phase $^{129}$Xe examined $^{129}$Xe dissolved in the blood and tissue of mice and rats, and proposed application in the measurement of lung function, kidney perfusion, myocardial perfusion and regional cerebral blood flow (Wagshul et al Magnetic Resonance in Medicine 1996, 36(2): 183-191; and Swanson et al Magnetic Resonance in Medicine 1999; 42: 1137-45). US 2001/0000727 discloses a method of magnetic resonance imaging wherein magnetic resonance signal data is obtained by application of a large flip angle to excite dissolved phase $^{129}$Xe in the vasculature. No precise starting point for obtaining signal data is defined in the method of US 2001/0000727.

There is therefore scope for an improved minimally invasive *in vivo* method of evaluating a patient to identify the underlying condition(s) so that appropriate treatments can be pursued and/or to evaluate the efficacy of therapeutic treatments administered to treat those conditions.

Summary of the Invention

The present invention uses polarized $^{129}$Xe to evaluate whether a subject has chronic heart failure or other respiratory, cardiopulmonary, or systemic impairments or conditions.

The present invention is defined by the appended claims

In certain embodiments, the evaluating step may consider the time constant associated with the time it takes the polarized gas to travel across the membrane structure and then enter the blood so that the signal strength increases therein after the destroying stop. In addition, the evaluating step may consider one or more of the oxygen saturation level, the ejection fraction, of the tissue volume based on the dynamic data.

In other embodiments, the subject can be evaluated both during exercise and when ar rest and/or after administration of a therapeutic agent to evaluate cardiopulmonary or pulmonary function and/or therapeutic efficacy.

As will be appreciated by those of skill in the art in light of the present disclosure, embodiments of the present invention may include methods, systems and/or computer program products. The foregoing and other objects and aspects of the present invention are explained in detail herein.

Brief Description of the Drawings

**Figure 1A** is a prior art enlarged micrograph of lung tissue and/or alveolar structure.
**Figure 1B** is a prior art greatly enlarged micrograph of lung tissue and alveolar structure illustrating the alveolar epithelium, the interstitium, the capillary endothelium, a capillary and red blood cells and alveolar air space.
**Figure 2A** is a NMR spectroscopic graph of polarized $^{129}$Xe peaks of interest according to the present invention.
**Figure 2B** is a simulated graph of the signal of the $^{129}$Xe blood component over time according to the present invention.
**Figure 2C** is a simulated graph of the signal of the $^{129}$Xe tissue component over time according to the present invention

**Figure 3** is a simulated graph of the uptake of polarized $^{129}$Xe in blood over time. The graph illustrates three different curves, each representing a different dynamic $^{129}$Xe behavior and/or alveolar-capillary membrane thickness that can be evaluated to ascertain information about the patient according to the present invention.

**Figure 4A** is a polarized gas $^{129}$Xe ventilation image of the lungs and **Figure 4B** is a graph of the polarized $^{129}$Xe uptake in blood over time; each can be generated based on a single-breath or ventilation administration of the polarized $^{129}$Xe according to enibodiments of the present invention.

**Figure 5** is a flow chart illustrating operations of a method, system, or computer program for spectroscopic analysis according to embodiments of the present invention.

Detailed Description of Embodiments of the Invention

The present invention will now be described more fully hereinafter with reference to the accompanying figures. This invention may, however, be embodied in many different forms and should not be construed as limited to the embodiments set forth herein. Like numbers refer to like elements throughout. In the figures, layers, regions or features may be exaggerated for clarity. Broken lines in the figures represent optional features or operations.

As known to those of skill in the art, polarized gases are collected, frozen, thawed, and used in MRI and NMR spectroscopy applications. For ease of description, the term "frozen polarized gas" means that the polarized gas has been frozen into a solid state. The term "liquid polarized gas" means that the polarized gas has been or is being liquefied into a liquid state. Although each term includes the word "gas", this word is used to name and descriptively track the gas that is produced via a hyperpolarizer to obtain a polarized "gas" product. Thus, as used herein, the term "gas" has been used in certain places to descriptively indicate a hyperpolarized noble gas product and may be used with modifiers such as solid, frozen, dissolved, and liquid to describe the state or phase of that product Also, for certain embodiments, the hyperpolarized gas is processed such that it is a pharmaceutical grade product suitable for *in vivo* delivery to a human subject- In particular embodiments, the $^{129}$Xe gas product is formulated to have less than about 10 ppb (parts per billion) alkali metal therein, and can have less than about 1 ppb.

Various techniques have been employed to accumulate and capture polarized gases. For example, U.S. Patent No. 5,642,625 to Cates et al., describes a high volume hyperpolarizer for spin-polarized noble gas and U.S. Patent No. 5,809,801 to Cates et al. describes a cryogenic accumulator for spin-polarized [129]Xe. U.S. Patent No. 6,079,213 to Driehuys et al., entitled "Methods of Collecting, Thawing, and Extending the Useful Life of Polarized Gases and Associated Apparatus", describes an improved accumulator and collection and thaw methods.

As used herein, the terms "hyperpolarize," "polarize," and the like, are used interchangeably and mean to artificially enhance the polarization of certain noble gas nuclei over the natural or equilibrium levels. Such an increase is desirable because it allows stronger imaging signals corresponding to better MRI images and spectroscopy signals of the gas in the body. *See* Albert et al., U.S. Patent No. 5,545,396. As is known by those of skill in the art, hyperpolarization can be induced by spin-exchange with an optically pumped alkali-metal vapor or alternatively by metastability exchange.

The present invention <u>is</u> directed to methods of evaluating a subject for respiratory disorders and/or cardiopulmonary disorders including chronic heart failure. Generally stated, polarized $^{129}$Xe can be administered to a subject in *vivo* and monitored to determine one or more of whether low oxygen saturation is the result of poor ventilation, poor perfusion, alveolar capillary membrane thickness, and/or poor gas diffusing capacity across the alveolar membrane, as well as pulmonary edema and the like. Such an analysis can be used to diagnose chronic heart failure, to differentiate uncertain aetiology of breathlessness or shortness of breath (or other breathing impairments) such as to identify cardiac or respiratory origin, to determine the adequacy of the alveolar-capillary unit, system or function, and to monitor therapeutic efficacy of treatments on those conditions.

Other potential clinical indications which can be assessed by methods of the present invention include, but are not limited to, the presence and/or extent of emphysema, the presence and/or extent of alveolitis (and follow-up monitoring for same), to differentiate pure airway conditions from conditions which affect the alveolar airspaces, the diagnosis of alveolar hemorrhagic conditions, and the diagnosis of radiation pneumonitis. In addition, the efficacy of therapeutics administered to treat a condition can be evaluated as a part of the treatment or as a part of pre-clinical drug trials to help establish the clinical efficacy of the proposed drug.

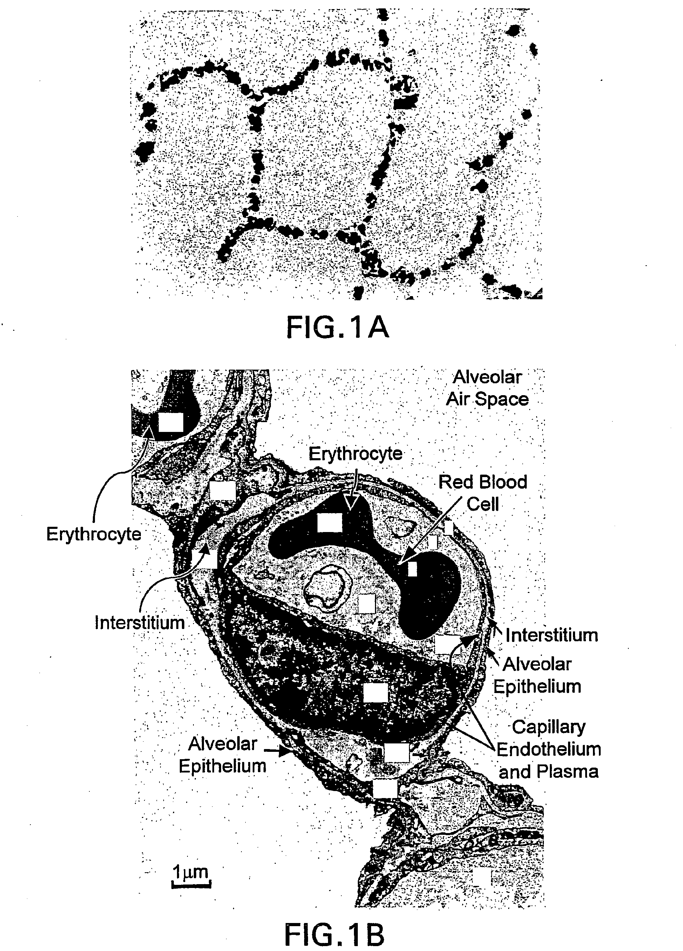

**Figure 1A** is a micrograph of lung tissue or alveolar structure. **Figure 1B** is enlarged (20X) compared to the scale shown in **Figure 1A** and illustrates alveolar structure including the alveolar epithelium (Ep), the interstitium (Ei), the capillary endothelium (En), a capillary (C) and red blood cell (R) and alveolar air space (A). The present invention provides methods for evaluating gas transit behavior across lung tissue, and/or across the alveolar-capillary membrane into the pulmonary blood. In certain embodiments, this information can be used to evaluate chronic heart failure ("CHF") because CHF typically disturbs the alveolar-capillary membrane and increases the resistance to polarized gas transfer as the gas attempts to travel toward the pulmonary blood (into the red blood cells). Elevation of the capillary pressure can increase the capillary permeability to water and ions and disrupt local regulatory mechanisms for gas exchange, leading to thickening of the alveolar-capillary interstitium, and/or to a decrease in membrane conductance and subsequent impairment of diffusion capacity. Monitoring of the transit time and/or behavior of $^{129}$Xe acting as a tracer across this membrane in resting conditions and during (incremental) actual or simulated exercise can be performed so as to measure the function of the alveolar-capillary membrane. Other physiology or structures (barriers) and associated peaks and regions can be monitored for other conditions as noted above.

In operation, in certain particular embodiments, a dynamic data set of NMR spectroscopic blood signal strength of polarized $^{129}$Xe in blood over time at at least one chemical peak or shift of interest can be obtained and evaluated to assess certain physiological parameters or function. The parameters can include, but are not limited to, one or more of perfusion uptake, the function of the alveolar-capillary membrane, and/or the thickness of the alveolar-capillary membrane, the alveolar transit time of the polarized gas, the oxygen saturation level or measure of ventilated blood volume (as a measure of shunt) in the patient. The dynamic data set corresponds to the accrual, build up, or increase in signal strength of a particular signal or signals with an associated chemical shift (such as in the pulmonary blood or alveolar tissue), over time.

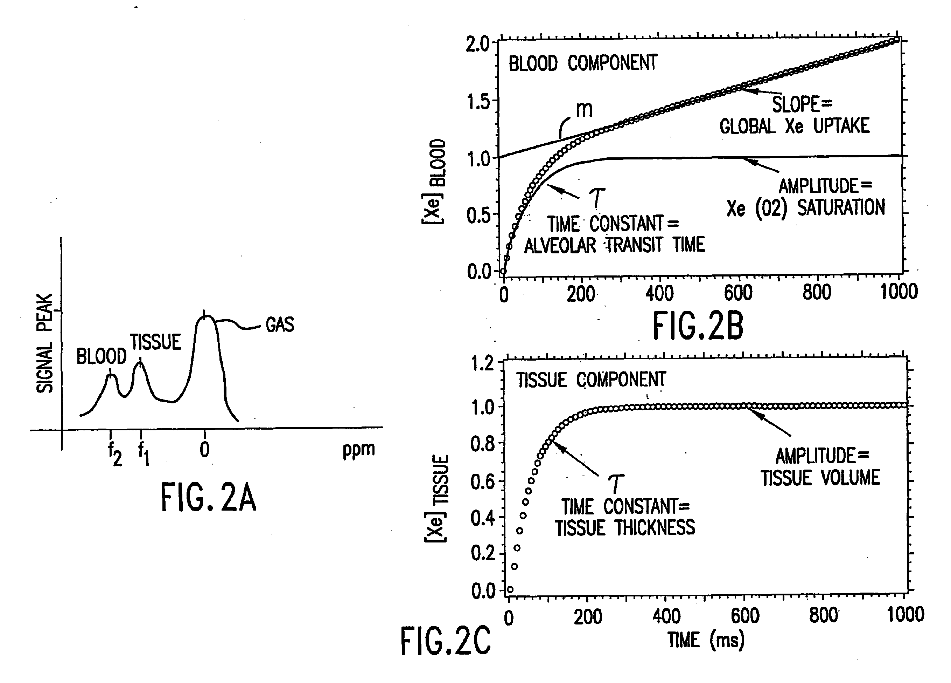

Referring now to **Figure 2A,** NMR signal strength spectrum data (obtained *in vivo*) of polarized $^{129}$Xe in the body of a subject is illustrated. The dissolved-phase spectra are shown on the left side of the figure (indicated by peaks at $f_1$ and $f_2$). The gas-phase spectra (on the right side of the figure at "0") have a larger signal relative to the spectra of the $^{129}$Xe dissolved in blood or tissue. When a quantity of polarized $^{129}$Xe is inhaled into the lungs, a small fraction of this gas (roughly about 0.3% per/second) transits into the pulmonary blood. It is known that polarized $^{129}$Xe in the lung exhibits three distinct NMR resonances: 0 parts per million ("ppm") is associated with gaseous $^{129}$Xe, 197 ppm is associated with $^{129}$Xe dissolved in lung tissue ($f_1$), and 212 ppm is associated with $^{129}$Xe dissolved in blood ($f_2$). Each of these resonances can be tracked as a function of time.

**Figure 2B** illustrates the signal strength of the $^{129}$Xe in the blood ($f_2$) over time and **Figure 2C** illustrates the signal strength of the $^{129}$Xe in the tissue ($f_1$) over time. To obtain the curve or line shape representing the gas exchange or diffusion process in the body at a selected frequency or shift of interest, a series of increasingly longer pulses are transmitted to generate the response signal. A plurality of different dynamic data sets, each at different discrete frequencies or chemical shifts can be obtained concurrently.

To generate the dynamic data set of the $^{129}$Xe in the body, polarized $^{129}$Xe is administered to the subject or patient. Then the polarization of the polarized $^{129}$Xe in a selected *in vivo* environment, structure, or physiology can be destroyed and allowed to rebuild. For example, gaseous polarized $^{129}$Xe can be inhaled into the lungs and then the $^{129}$Xe can diffuse to, and hence supply, the polarized $^{129}$Xe into the membrane (as it diffuses serially across Ep, Ei, and then En) and into the capillary and the pulmonary blood.

As shown in **Figure 2B,** the build up of signal of the polarized $^{129}$Xe in blood can be dynamically monitored to determine a time constant "τ" associated with transit time of the polarized $^{129}$Xe as it diffuses across the alveolar-capillary membrane. In operation, in certain embodiments, to measure the time constant or transit time, the polarization, and hence the signal, in the alveolar-capillary membrane (which can include the alveolar epithelium, the interstitium, and the capillary endothelium) and proximate blood in the capillary/red blood cell(s) can be destroyed by transmitting a large angle RF excitation pulse thereto to define t=0 (defined as the time after the polarization is destroyed). Then NMR signal strength data of the polarized $^{129}$Xe is collected for a first time period. The signal strength of the chemical shift spectra of the polarized gas entering the blood increases over time as it rebuilds from "0" from the ongoing supply of polarized gas delivered from the lungs during the analysis period.

Then the polarization is destroyed again and a second data collection is commenced by transmitting a second excitation pulse that is initiated at a second time (delayed relative to the first excitation to obtain data associated with the polarized $^{129}$Xe at a period that is later in time from the first excitation), such as at t=40ms. Thus, to generate the line or curve (at least electronically) associated with the dynamic data set, the polarization is destroyed a plurality of times and then a series of incrementally spaced excitation pulses are transmitted to generate a response curve which maps the $^{129}$Xe behavior *in vivo* with at least about millisecond resolution. Stated differently, the polarization is destroyed each time and then the response data is collected at successively longer times to collect the signal data at the desired times in the analysis period. The NMR data can be collected using decremented pulse intervals (starting with the longest interval and moving to the shortest) or other sequences as desired. The data can be collected and the response curve of the $^{129}$Xe can be generated using curve fit using statistical analysis techniques well known to those of skill in the art. Other peaks at other locations, environments, tissues or membranes in the body may be selected in other embodiments and similarly evaluated for function.

**Figure 2C** illustrates that the dynamic data can be generated with sufficient resolution to be able to measure uptake with sufficient resolution to define a line shape corresponding to uptake of the $^{129}$Xe. To obtain sufficient data points to generate the time constant "τ", the signal data can be collected for times that are at least two times that of the time constant. Thus, for time constants of 60ms, data can be collected for at least about 120ms. In certain embodiments, the time constant "τ" can be used to determine the thickness of the alveolar-capillary membrane.

As shown in **Figure 2B,** in certain embodiments, after inhalation of hyperpolarized $^{129}$Xe, its uptake into the pulmonary blood can be measured as a function of time. The initial build-up rate (after the polarization is substantially destroyed in the membrane and the blood) of the xenon/blood signal is sensitive to alveolar membrane thickness. The spectroscopic signal of the xenon/blood can be used to quantify the thickness and/or the degree of pulmonary edema or the function or thickness of the alveolar-capillary membrane associated with various cardiopulmonary conditions such as chronic heart failure. The quantified or estimated thickness of the membrane and/or the initial data associated with the gas exchange during the initial build up can also be used to evaluate other conditions such as measuring/quantifying pulmonary fibrosis or other respiratory conditions as noted above.

In other embodiments, the response or behavior of the $^{129}$Xe during other portions of the analysis period can be used to evaluate total cardiac output and/or ventilation/perfusion ratios. For example, the curve or line shown can be broken down into at least three components: the slope "m" corresponding to the linear rise in the signal after the initial portion of the signal, the time constant "τ" associated with the initial portion of the signal, and the amplitude defined by the a steady state amplitude of the signal deconvoluted from the data representing the signal at the t>> than the calculated time constant "τ". In particular embodiments, the amplitude of the signal that is 37% greater than the amplitude of the signal at the time=τ. The slope "m" can be used as a measure of global xenon uptake (as a tracer for oxygen) in the blood. The slope of this curve ("m") may yield information about global blood flow to the ventilated portion of the lung and, hence, may be a predictor of shunt or cardiac output. Similarly, the amplitude ot the $^{129}$Xe spectroscopic signal defined in relationship to the time constant can be used as a measure of oxygen saturation (ventilated blood volume). The time constant "τ" can also be used to evaluate or determine the thickness, function, or physiology of the alveolar-capillary membrane.

In other embodiments, as shown in **Figure 2C,** the NMR signal in tissue can also be monitored to evaluate tissue volume (corresponding to the peak amplitude of the curve associated therewith) while the time constant "τ" associated with the signal in tissue can be used as a measure of tissue thickness.

The NMR signals and associated evaluations and measurements can be performed while the subject is exposed to actual or simulated exercise.

In addition, in certain embodiments, the $^{129}$Xe inhalation can be used to monitor an administered therapy for efficacy or for drug development processes where a new drug or use is undergoing evaluation during clinical or pre-clinical trials.

Measurement of the xenon/blood resonance signal (S) or the xenon/tissue resonance as a function of time ("S (t))" can be expressed by the following mathematical relationship:

$$S(t)=S_0(1-e^{-t/\tau}). \hspace{4cm} \text{Equation (1)}$$

Where "τ" is the time constant for polarized $^{129}$Xe associated with the uptake and/or dissolved gas-exchange transit time, and "S" is the signal strength of the polarized $^{129}$Xe at the selected frequency (or in the bio-environment or bio-structure of interest). These time constants have been measured in dogs to be at about 61ms and 70ms for the tissue and blood compartments, respectively. *See* Ruppert et al., NMR of Hyperpolarized 129Xe in the Canine Chest: Spectral Dynamics During a Breath-Hold, 13 NMR in Biomediciene, p. 623-641 (2000). The time constants are representative of the amount of time it takes xenon to diffuse across the alveolar membrane and into the red blood cells. The diffusion constant of xenon in water is about $2 \times 10^{-5}$ cm$^2$/s. *See* Wolber et al., Measuring Diffusion of Xenon in solution with hyperpolarized 129Xe NMR, 296 Chemical Physics Letters, p. 391-396, (Nov. 6, 1998). The mean square distance traveled by a randomly diffusing gas can be approximated as described by Equation (2).

$$Z^2=2Dt. \hspace{5cm} \text{Equation (2)}$$

Where "$Z^2$" is the mean square distance, "D" is the diffusion coefficient constant and "t" is the time it takes the gas to diffuse through the membrane. Thus, from the two transit times measured above, mean thickness or diffusion distances of about 15.6 μm and about 16.7 μm can be calculated. It is noted that these curves are taken from the entire lung and include regions where tissue is thicker or thinner. Alternative pulse sequences can be used to identify the uptake in the first few milliseconds (the transmit times are typically under about 100ms) corresponding to transit times across the thinnest membranes. At the onset of certain diseases, the mean alveolar transit time can become longer. Because diffusion time is proportional to the square of the tissue thickness, the transit time will be sensitive for quantifying wall thickness and/or thickening (or thinning as the case may be).

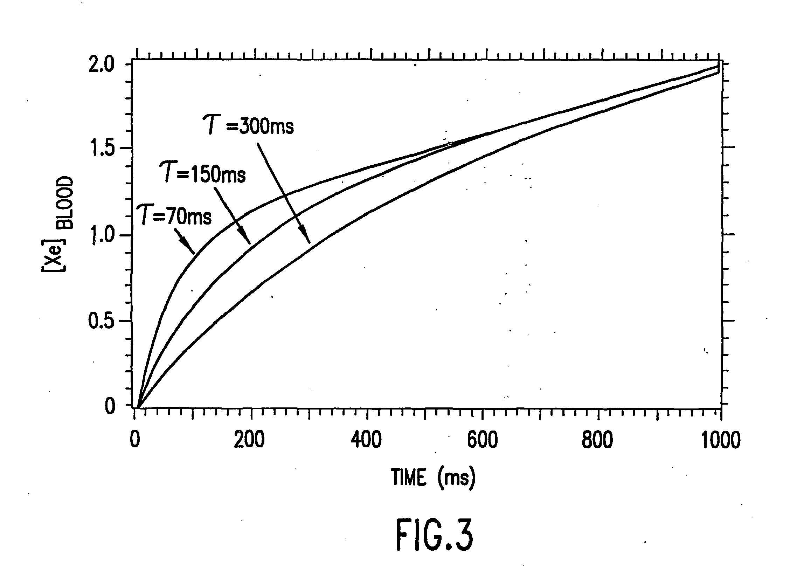

**FIGURE 3** illustrates graphs of curves having three different time constants for the polarized $^{129}$Xe signal in blood illustrating the cardiopulmonary functional change that may be representative of heart failure or other disorders, diseases, or conditions. The time constant can be calculated by Equation 1 and is typically defined at when the signal is at about 63% of its ultimate value. As shown, the time constant for each of the curves varies in a quantifiable manner. The longer time constants (*i.e.,* $\tau$=150 and 300ms) correspond to thicker alveolar-capillary membranes. In certain diseases or conditions, the alveolar membrane can thicken in response to hypertension or other conditions. As the condition deteriorates, the transit time or time constant "$\tau$" increases (shown as going from 70ms to 300 ms) corresponding to the thickening of the alveolar membranes in response to hypertension and the like (reducing oxygen diffusing capability). Many therapeutic regimens attempt to thin the alveolar membranes and the present invention can assess whether this objective has been achieved by evaluating the time constant $\tau$ and/or other $^{129}$Xe signal parameter. As many therapies attempt to cause the alveolar membrane to thin, monitoring such a progression or behavior to confirm that the therapy is effective of that the condition is not deteriorating may provide important clinical information.

The signal strength of the dissolved phase polarized xenon signal intensity versus repetition time will have an associated slope "m" which is a function of the signal and the pulse repetition ($dS_p/dT_R$). In operation, a large flip angle pulse (preferably a flip angle of about 90 degrees) can be transmitted to the blood; this destroys all the magnetization in the xenon and, thus, the signal of the dissolved polarized xenon in the blood. Subsequently, after the excitation pulse, additional polarized $^{129}$Xe is taken-up in the blood (replenished) over time until a substantially steady state level is reached: the more polarized xenon in the blood, the larger or stronger the associated signal. This increase in the dissolved phase xenon signal over time (after the initial transit time across the barrier ) can be mathematically represented by the slope of the line ($dS_p/dT_R$). The slope (after an initial gas blood barrier crossing period) can be directly proportional to blood flow rate (Q) in the bloodstream.

In order to determine the slope of the line associated with the signal of the dissolved phase xenon in the blood over time, the data acquisition can obtain several data points such as three-ten temporally separate data points within the first 60ms to establish the time constant associated with the curve fit of the signal shape/$^{129}$Xe behavior at the peak (s) of interest. In certain embodiments, it will take longer for blood to uptake polarized xenon where there is low blood flow in ventilated regions in the lung and a shallower blood signal slope may be indicated (representing a low blood flow rate through the ventilated regions of the lung).

In certain embodiments, the slope data of the dissolved phase polarized $^{129}$Xe in tissue and/or blood versus can be adjusted by comparing it to the gas phase signal in the lung ($S_L$). This gas phase signal ($S_L$) is acquired from an excitation signal with a known flip angle $\forall_L$ (the polarized gas is conveniently available in the lung space). Thus, the present invention can use a mathematical relationship between the dissolved phase polarized xenon signal in the blood or tissue with the xenon signal in the gaseous phase in the lung to establish a quantitative measure of signal.

In particular embodiments, a lung volume ($V_L$) is measured by conventional means before or after the MR procedures, or by assuming an average or normalized lung volume for a particular patient size or age. After a short initial time, the slope of the curve corresponds to the blood flow rate in the blood stream.

In summary, according to certain embodiments of the present invention, there are several quantifiable parameters that can be derived from the $^{129}$Xe uptake spectra: Speak (tissue), $\tau$ (tissue), $S_{peak}$(blood), $\tau$ (blood), and slope of the linear uptake portion of the xenon/blood resonance. These uptake spectra may be performed on a regional basis in the lung. This dynamic signal data can be obtained with millisecond or better resolution. In certain embodiments, the alveolar transit time, oxygen saturation level, global perfusion, tissue volume and ejection fraction can be evaluated to identify any abnormalities or alterations in physiology or function.

Generally stated, the ventilated blood flows to the heart to the left atrium to the left ventricle and pumped to body through the (arch of) aorta. The blood is forced or ejected from the heart in pulsatile flow corresponding to the pumping action thereof. The pulsatile flow behavior of the blood ejected from the aorta can be monitored to evaluate or map the ejection fraction. Gradient-tagged RF excitation pulses can be used to look at the signal of the $^{129}$Xe in the blood as it exits the aorta or left ventricle (or region proximate thereto). This targeted region can be monitored to obtain the signal strength of the $^{129}$Xe in this ejected blood over time, the signal will increase and decrease corresponding to the cardiac cycle and the signal can be evaluated to assess how much of the polarized blood is pumped out of the left ventricle or aorta in each pumping cycle. This ejection fraction can be compared to the subject's own previous evaluation or based on a statistical population average (by gender and/or age which can be generally stated to be an average of about 60%) to asses whether there is an abnormality. Thus, for example, if 200ml of ventilated (polarized) blood is pumped into the heart and 100ml is ejected, the ejection fraction may be identified as 50% (lower than average). Thus, in certain embodiments, the dynamic gas exchange data of signal over time in one, two, or more different environments, regions, or tissues (such as tissue and blood) can provide information on ventilation, perfusion, and ejection fraction, as noted above.

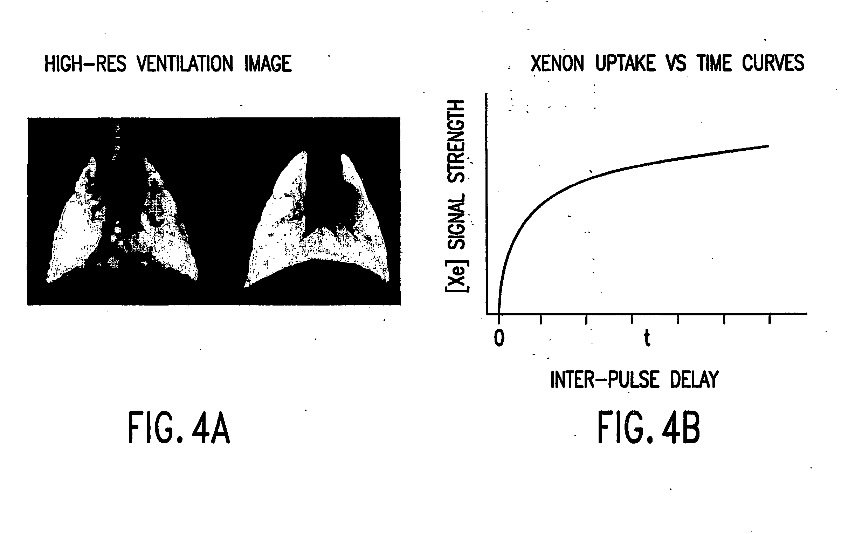

In addition, as shown in **Figures 4A** and **4B,** in certain embodiments, there may be enough magnetization or polarization associated with the $^{129}$Xe *in vivo* to perform a polarized $^{129}$Xe ventilation or $^1$H MRI ventilation image in the same breath-hold period, typically of about 10 seconds. The combination image/scan with the spectroscopic analysis can provide additional information on the state of the cardiopulmonary system. In certain embodiments, the test can be carried out in an MRI magnet with a dual-tuned $^{129}$Xe/$^1$H coil to allow conventional or standard imaging to be performed to yield anatomical information in the same MR imaging/spectroscopy session. It is noted that the image shown is based

on polarized $^{3}$He because it is readily accessible, but it is anticipated that a similar resolution ventilation image can be generated using polarized $^{129}$Xe.

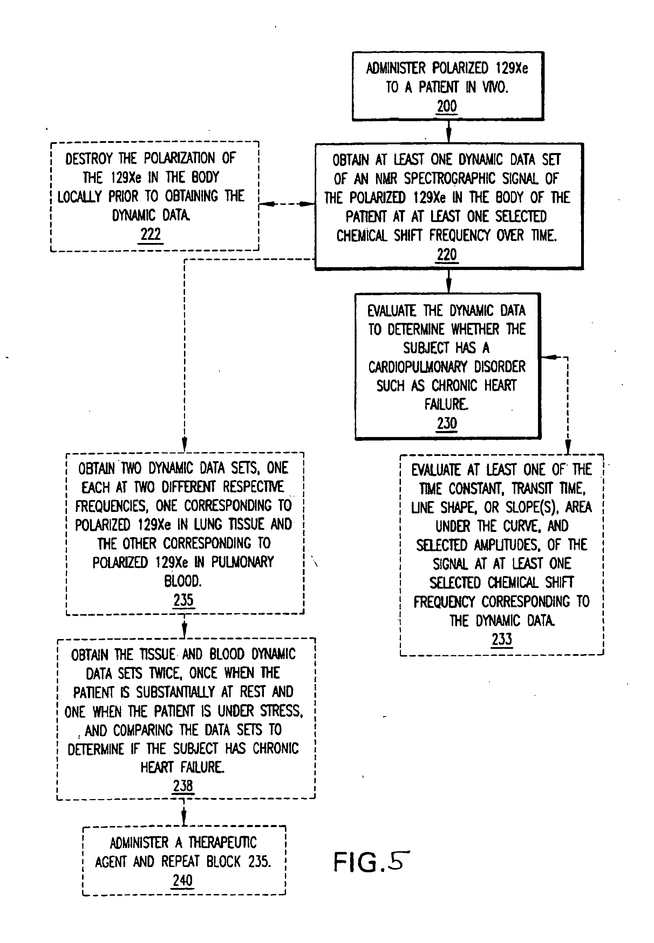

**Figure 5** is a block diagram of a method for assessing whether a subject has CHF. As shown, polarized $^{129}$Xe is administered to the subject **(Block 200)**. At least one dynamic da ta set is obtained of an NMR spectrographic signal of the polarized $^{129}$Xe in the body at at least one selected chemical shift frequency over time **(Block 220)**. The polarization level of the $^{129}$Xe in the body can be destroyed locally prior to obtaining the dynamic data **(Block 222)**. The dynamic data set can be evaluated to assess the polarized gas behavior proximate the alveolar-capillary membrane *in vivo* to determine whether the patient has CHF **(Block 230)**. In certain embodiments, at least one parameter of interest is evaluated based on a curve fit to the dynamic data set: the parameters can include the time constant of the signal, the transit time of the polarized $^{129}$Xe, the line or curve shape of the signal, the area under the curve, and the amplitude (at the time constant or peak) **(Block 233)**.

In particular embodiments, two dynamic data sets, one each at two different respective frequencies can be obtained, one corresponding to polarized $^{129}$Xe in lung tissue, the other corresponding to polarized $^{129}$Xe in pulmonary blood **(Block 235)**. In addition, the two data sets can be obtained twice, once when the patient is substantially at rest and one when the patient is exposed to actual or simulated exercise and comparing the data sets to determine if the subject has chronic heart failure **(Block 238)**. Further, a therapeutic agent can be administered and the operation in Blocks 235 and/or 238 can be repeated **(Block 240)**.

Generally stated, in operation, a patient is positioned in an MRI unit and exposed to a magnetic field. The MRI unit typically includes a super-conducting magnet, gradient ceils (with associated power supplies), a NMR coil (transmit/ receive RF coil), and a RF amplifier for generating RF pulses set at predetermined frequencies. For $^{129}$Xe imaging at 1.5T field strength, the MRI unit is set to operate in the gas-phase at about 17.6 MHz. The dissolved phase excitation frequency is shifted below the gas phase excitation frequency such as about 196 to at least 200 ppm lower than the gas phase excitation frequency (corresponding to the chemical shift). Thus, the dissolved phase $^{129}$Xe RF excitation frequency can be about 3.52kHz lower than the associated gas-phase excitation frequency. In other embodiments, the imaging method employs a 17.6MHz gas phase excitation pulse and an associated dissolved phase excitation pulse of about 17.59648MHz- Of course, the magnet field strength and excitation frequency can vary as is well known to those of skill in the art depending on the target region/tissue or environment undergoing evaluation.

In any event, the RF pulse(s) is transmitted to the patient to excite the nuclei of the polarized $^{129}$Xe. The NMR coil is tuned to a selected frequency range and positioned adjacent the targeted imaging region to transmit the excitation pulses and to detect response to the pulse sequence generated by the MRI unit. NMR coils for standard chest imaging can include a wrap-around coil with conductors positioned on both the front and back of the chest. Examples of acceptable coils known to those of skill in the art include a bird-cage configuration, a Helmholtz pair, a NMR coil, and a solenoid coil (for permanent magnets). Other NMR coils can be used for other imaging regions of the body (such as the head, torso, and the like).

In certain embodiments, the patient can inhale a quantity of polarized $^{129}$Xe gas into the pulmonary region (*i.e.*, lungs and tracbea)- After inhalation, the patient can hold his or her breath for a predetermined time such as 5-20 seconds. This can be described as a "breath-bold" delivery. Examples of suitable "single dose" quantities of polarized gases for breath-hold delivery include 0.25-0.5, 0.75, and 1.0-2.0 liters of gas. The dose at inhalation can contain gas with a suitable polarization level, typically so that the polarization at delivery is well above 5%, and preferably a polarization level above about 20%-50%.

As used herein, "large flip angle" means an angle that is greater than about 30 degrees, and typically greater than about 75 degrees, and more typically about 90 degrees. A 30-degree flip angle will generally yield about 50% as much signal as a 90-degree flip (45 degrees typically giving about 70% as much signal).

An exemplary delivery of a selective excitation pulse is via a "hard" pulse- As used herein, "hard" pulse includes pulses where the RF is turned on for a short pulse time ("$t_{pulse}$") and then shortly thereafter, indeed preferably substantially "instantly," turned off. However, short pulse times can yield uncertainty in the associated frequency it generates- In certain embodiments, selective excitation is performed such that the pulse frequency is centered on the dissolved gas phrase resonance desired (*i.e.*, 17.59648 MHz) and has a pulse time, $t_{pulse}$, such that the associated frequency is below the corresponding gas phase excitation frequency (*i.e.*, 17.6 MHz). For example, one frequency spectrum of a square excitation pulse having a time $t_{pulse}$ and which is centered on a frequency ("fo") can be described by the equation:

$$\sin(a(f\text{-}fo)/a(f\text{-}fo)), \text{ where } a = 3.1416{*}t_{pulse}. \qquad \text{(Equation 3)}$$

Therefore, the pulse time $t_{pulse}$ is preferably set so that the sin (a(f-fo))=O for the gas phase component. Stated differently, the pulse time $t_{pulse}$ is determined according to the relationship $t_{pulse}$ =1/(f-fo). In one embodiment, for a 1.5T magnetic field strength, f-fo equals 3.52kHz and $t_{pulse}$ is about 284 µseconds ($10^{-6}$). Of course, as will be recognized by those of skill in the art, alternative approaches can also be used, such as, but not limited to, sine pulses, gaussian pulses, and the like.

In the present invention, a large flip angle pulse is delivered to the target region so as to substantially destroy the incoming $^{129}$Xe polarization or magnetization to set the "0" or monitoring start window for obtaining data at successively longer pulse delay times to analyze the transit time and/or gas exchange dynamics. Thereafter, in certain embodiments, the selective excitation is timed such that it excites the entire pulmonary blood volume. The pulmonary blood volume includes the volume of blood that fills the blood passages associated with the circulatory system between and/or within the lungs and the heart (which can include the volume of blood or a portion of the volume of blood within the boundary lung tissue and/or heart). Advantageously, unlike imaging the gas-phase $^{129}$Xe in the lung where conventionally small flip angles are used to avoid destroying the available magnetization, a large flip angle excitation of the dissolved phase $^{129}$Xe in the pulmonary vasculature allows for the initialization of the "0" level to monitor the gas-exchange dynamics. Further, according to the certain embodiments using inhalation delivery of the $^{129}$Xe, "fresh" magnetization (*i.e.*, polarized $^{129}$Xe) is substantially continuously flowing in from the capillary beds during the procedure. See coassigned and co-pending U.S. Patent Application Serial No. 09/271,476 and U.S. Patent Application Serial No. 09/271,476 for descriptions of imaging methods using $^{129}$Xe.

The term "pulmonary and cardiac vasculature" as used herein includes all of the blood vessels within the lungs and/or heart, the chambers of the heart, the passages between the chambers of the heart, as well as the blood vessels between the lungs and heart, and blood vessels between the lungs or heart and other tissues and/or organs. The pulmonary and cardiac vasculature includes, but is not limited to, the pulmonary veins and arteries and associated capillaries, the left and right atria of the heart, the left and right ventricles of the heart, the myocardium, the aorta and aortic arch, the coronary artery, the coronary arteries, the subclavian arteries, and the carotid arteries.

Almost immediately upon inhalation of hyperpolarized $^{129}$Xe into the lungs, Xe begins to dissolve into the pulmonary blood stream (typically in under about 100ms). The concentration of Xe in the pulmonary capillary beds ("[Xe]$_p$"), can be assumed to equilibrate after an initial gas transit time (as the gas travels across the alveolar-capillary membrane) with the concentration of Xe in the lung gas spaces ("[Xe]$_L$"). Thus, the relationship can be stated as:

$$[Xe]_P = 8\ [Xe]_L \qquad , \qquad \text{(Equation 4)}$$

where "8" is the Xe blood/gas partition coefficient or blood solubility. This concentration can be expected to equilibrate in the venous side of the pulmonary vasculature just a few seconds after inhalation. The standard unit for concentration is an "amagat" which refers to 1 atmosphere of gas pressure at a temperature of 273K. For humans whose lungs contain one atmosphere of gas and whose temperature is about 310K, all gas densities should be scaled down by a factor of about A=0.88 amagat per atmosphere. For a patient inhaling a volume ("$V_{Xe}$") of Xe into their lungs of volume (" $V_L$"), the resulting Xe density in the lung [Xe]$_L$ will be

$$[Xe]_L = A \frac{V_{Xe}}{V_L} \qquad . \qquad (Equation\ 5)$$

Thus, the concentration of Xe in the pulmonary blood [Xe]$_P$ will be related to the inhaled gas volume $V_{Xe}$, and can be stated by the expression:

$$[Xe]_P = \lambda A \frac{V_{Xe}}{V_L} \qquad . \qquad (Equation\ 6)$$

For reference, an estimate of λ for Xe in blood is that 8 ≈ 0.15. Thus, as an example, a patient who inhales 1L of Xe into his 6L lung will yield a Xe density in the lungs of [Xe]$_L$ ≈ 0.15 amagat, and correspondingly a Xe density in the pulmonary capillary beds of [Xe]$_P$ ≈ 0.02 amagat. Thus, the dissolved polarized $^{129}$Xe gas in the pulmonary capillary beds will substantially saturate at approximately 1/6 the concentration of the lung gas.

As described above and in co-pending U.S. Patent Application Serial No. 09/271,476, a patient who inhales 1L of Xe into the lungs (having about a 6 L lung volume) will yield about or dissolve into or saturate at about 1/6 of that value of the xenon concentration (.02 amagat) in the pulmonary vasculature and associated blood. For additional description of signal compensation or adjustment, signal per voxel, and perfusion, images, *see* U.S. Patent Application Serial No. 09/271,476. In certain embodiments, the method uses frequency selective large angle (more preferably 90°) RF excitation pulses that substantially depletes the $^{129}$Xe in the pulmonary blood but leaves the hyperpolarized gas in the lungs substantially undisturbed to define the initial monitoring period during which dynamic NMR signal data is obtained. In this embodiment, the repetition time interval between RF pulses ($T_R$) and the pulmonary blood flow rate (Q) can be used to determine the effective pulmonary volume ($V_{eff}$) containing (dissolved phase) hyperpolarized $^{129}$Xe. This relationship assumes that $T_R$ is less than or substantially equal to the time it takes for the polarized $^{129}$Xe to leave the pulmonary blood (tp). As discussed above, for typical blood flow rate and estimated volume of venous pulmonary blood, $t_p$ is approximately 2.5 seconds. Thus, with a large RF excitation pulse (preferably, about $\alpha=90°$), the dissolved pulmonary $^{129}$Xe signal strength in the pulmonary blood is proportional to the product of coil gain ("G"), Xe polarization ("$P_{xe}$"), and polarized Xe density or concentration in the vasculature ($[Xe]_P=\lambda[Xe]_L$), which can be stated by the following expression:

$$Sp(T_R)=GP_{Xe}8[Xe]_L\,QT_R \qquad\qquad . \qquad\qquad \text{Equation (7)}$$

Notably, the signal strength is dependent on both the pulse interval ($T_R$) and the blood flow rate (Q). The dissolved signal intensity versus repetition time will have an associated slope which can be mathematically expressed as follows:

$$\frac{dS_P}{dT_R} = GP_{Xe}\lambda[Xe]_L Q \qquad\qquad . \qquad\qquad \textit{Equation (8)}$$

The slope "m" of polarized xenon in the pulmonary blood is directly proportional to the pulmonary blood flow rate (Q). Calibration of the blood flow rate is obtainable by evaluating the gas phase signal ("$S_L$") in the lung, the signal having an associated small RF tipping angle (excitation angle) ("$\alpha_L$"). The gas phase signal can be expressed by the equation:

$$S_L = GP_{Xe}[Xe]_L V_L \sin\alpha_L \qquad\qquad . \qquad\qquad \textit{Equation (9)}$$

The pulmonary blood flow rate (Q) can be stated by the ratio of the hyperpolarized $^{129}$Xe gas and dissolved phase signals. This ratio cancels receiver gain (G) and polarization value $P_{xe}$. Accordingly, the blood flow rate (Q) can be expressed by the following:

$$Q = \frac{V_L \sin\alpha_L\,(dS_P/dT_R)}{\lambda S_L} \qquad\qquad . \qquad\qquad \textit{Equation (10)}$$

Advantageously, with measurements of the Xe/blood partition coefficient ($\lambda$) and the total lung volume ($V_L$), a quantitative measurement of blood flow is established according to a method of the instant invention. As will be appreciated by one of skill in the art, lung volume can be easily established to about 20% accuracy with techniques known to those of skill in the art. Preferably, techniques with relatively improved accuracy such as but not limited to spirometry are used.

The spectroscopic evaluation methods do not require a polarization calibration because the measurement can be "self-calibrating." Stated differently, the polarization can be cancelled by comparing dissolved and gaseous xenon signal, both of which can be assumed to have substantially the same or identical polarization to the extent that T1 relaxation in the blood can be negligible.

The present invention has been described above with respect to particular preferred embodiments. Those skilled in the art, however, will appreciate that the invention can be employed for a broad range of applications. Methods for imaging or obtaining information about gas exchange barriers, physiologic function and dynamic functional evaluation of systems, membranes, biostructures or environments and/or perfusion mapping using dissolved hyperpolarized $^{129}$Xe can be carried out according to the present invention using magnetic resonance or spectroscopic techniques known to those skilled in the art. *See, e.g.,* U.S. Patent No. 5,833,947; U.S. Patent No. 5,522,390; U.S. Patent No. 5,509,412' U.S. Patent No. 5,494,655, U.S. Patent No. 5,352,979; and U.S. Patent No. 5,190,744. *See also* Hou et al., Optimization of Fast Acquisition Methods for Whole-Brain Relative Cerebral Blood Volume (rCBV) Mapping with Susceptibility Contrast Agents, 9 J. Magnetic Resonance Imaging 233 (1999); Simonsen et al., CBF and CBV Measurements by USPIO Bolus Tracking: Reproducibility and Comparison with Gd-Based Values, 9 J. Magnetic Resonance Imaging 342 (1999); Mugler III et al., MR Imaging and Spectroscopy Using Hyperpolarized 129Xe gas: Preliminary Human Results, 37 Magnetic Resonance in Medicine, pp. 809-815 (1997); Belliveau et al., Functional Cerebral Imaging by Susceptibility-Contrast NMR, 14 Magnetic Resonance in Medicine 14 538 (1990); Detre et al., Measurement of Regional Cerebral Blood Flow in Cat Brain Using Intracarotid 2H20 and 2H NMR Imaging, 14 Magnetic Resonance in Medicine 389 (1990); Frank et al., Dynamic Dysprosium-DTPA-BMA Enhanced MRI of the Occipital Cortex; Functional Imaging in Visually Impaired Monkeys by PET and MRI (Abstract), Ninth Annual Scientific Meeting and Exhibition of the Society of Magnetic Resonance In Medicine (August 18-24, 1990);Le Bihan, *Magnetic Resonance Imaging of Perfusion,* 14 Magnetic Resonance in Medicine 283 (1990); and Rosen et al., Perfusion Imaging by Nuclear Magnetic Resonance, 5 Magnetic Resonance Quarterly 263 (1989).

In particular embodiments, the present invention can be practiced to give a quantitative assessment of perfusion which can be used to evaluate systemic function as will be appreciated by one of skill in the art. According to this embodiment, signal intensity can be followed over time as noted above. Examples of such quantitative relationships were developed for use with radioactive contrast agents with MR imaging and spectroscopy methods may be particularly suitable for dissolved phase $^{129}$Xe analysis of blood vessels. *See, generally,* Lassen, Cerebral Transit of an Intravascular Tracer may Allow Measurement of regional Blood Volume but flot Regional Blood Flow, 4 J. Cores. Blood Flow and Metab. 633 (1984).

Furthermore, the inventive methods may be used for wide range of diagnostic and evaluative applications, preferably those related to cardiac, pulmonary or cardiovascular function, as described in more detail below. Other applications of the present invention include, but are not limited to: identification and assessment of the presence or absence and/or severity of cardiac ischemias and/or infarcts; localization and assessment of thrombi and plaques; determination of therapeutic windows" for administering heparin, vasodilators, antihypertensive agents, calcium antagonists and the like, *e.g.,* in reversible focal ischemia; monitoring of other induced vasodilator effects; detection and quantitative evaluation of the severity of ischemias; monitoring the vasodilatory or vasocontractory effects of a physiologically active substance; and monitoring surgically induced blood perfusion variations.

Many researchers have investigated characteristic chemical shifts observed when hyperpolarized $^{129}$Xe comes into contact with different tissues, as seen in Table 1. As shown, large frequency shifts (on the order of 200 parts per million or "ppm") from free gas phase (referenced at 0 ppm) have been observed. This frequency shift is far greater than that observed with proton spectroscopy (generally stated, at most about 5 ppm). Therefore, spectroscopy is a modality which may be particularly suited to capitalize upon the behavior of hyperpolarized $^{129}$Xe.

**Table 1.** Characteristic shifts from free gaseous hyperpolarized $^{129}$Xe (referenced at 0 ppm) of hyperpolarized $^{129}$Xe when exposed to different tissues.

| Tissue | ppm | Reference |
|---|---|---|
| Water | 191.2 | Wilson 99 |
| Epicardial fat | 192 | Swanson 99 |
| Brain lipid rich | 194 | Albert 99 |
| Brain tissue | 194.5 | Swanson 97 |
| Plasma | 195.6 | Wilson 99 |
| Brain | 198.0 | Wilson 99 |
| Lung parenchyma | 198.6 | Wilson 99 |
| Brain tissue | 199 | Swanson 99 |
| Kidney | 199.8 | Wilson 99 |
| Brain - lipid poor | 201 | Albert 99 |

(continued)

| Tissue | ppm | Reference |
| --- | --- | --- |
| Liver | 201.8 | Wilson 99 |
| *T. Californica* membrane | 209 | Miller 81 |
| RBC (oxygenated) | 213.0 | Wilson 99 |
| RBC (de-oxygenated) | 216.0 | Albert 99 |

As discussed hereinabove, hyperpolarized $^{129}$Xe can be administered to a patient by inhalation or injection. If the administration modality is injection, $^{129}$Xe can be, suspended in a carrier fluid or injected directly such as in gaseous form. However, regardless of what tissue is of interest, if the $^{129}$Xe is suspended in a carrier fluid, it is likely that the carrier fluid itself distorts the results of the spectra and/or substantially obscures a spectral peak of interest. The carrier fluid may also react with the target tissue (region of interest) and/or potentially produce compounds with molecules in or around the tissue of interest, which may thereby cause the chemical shift of hyperpolarized $^{129}$Xe to differ from that which would be observed with merely the tissue of interest and hyperpolarized [129]Xe. Therefore, direct injection of gaseous $^{129}$Xe or administration via inhalation may be particularly suitable for certain embodiments or applications. For additional discussion of direct injection of gaseous $^{129}$Xe, *see* co-pending U.S. Application Serial No. Serial No. 09/804,369.

In certain embodiments, the spectral peaks may be quantified by normalizing the spectral data. The term "normalizing" means to adjust the signal data of the spectral peak or peaks of interest to account for selected signal variables. This adjustment may include using the mathematic ratio of the values of certain peaks associated with selected known biomatter (RBC, plasma, etc) within the response spectrum to quantify the hyperpolarized gas signal in the region of interest. The adjustment may include using the polarization level (and/or quantity) of the administered gas measured at the time of deiivery to obtain a base or reference spectrum to quantify the magnitude of the signal. As such, the normalization can use relative data and/or absolute data. For example, the ratio of the spectra for the blood to spectra of the brain tissue (the ratio of the magnitude or area of selected spectral peaks) can be calculated. Of course, other known chemical shift peak locations can also be used to normalize the value of the spectra peak of interest. The absolute data can include data associated with the polarization level of the gas as it is delivered to the patient and/or the amount of gas administered thereto (to account for signal strength).

A region-specific NMR coil can be positioned over the region of interest and to transmit a selected Repulse sequence. The coil receives a FID signal. Localizing gradients can also be applied about the region of interest so as to localize the resonance region. For example, localizing gradients can be applied so that a desired region of interest is excited (either the left or right). In any event, the Fourier Transform of the acquired data is then calculated. The transformed signal data can be further processed, which processing may include, but is not limited to, one or more of subtracting background noise, filtering undesirable signal data (such as those portions of the signal or spectra attributed to carrier liquids or deposits in non-target tissue or blood and the like), determining the frequency shift and size of the shift for any number of peaks within predetermined ranges in the spectrum, and normalizing the data such as finding the ratios between magnitudes and/or areas of different spectral peaks within the response spectrum or accounting for polarization level and amount of polarized gas delivered to the subject. For further discussion of exemplary background subtraction or adjustment methods and cardiac gating methods, see co-pending U.S. Application Serial Nos. 09/271,476 and 09/271,476.

The present invention finds use for both pre-clinical animal studies, veterinary and medical applications. The present invention may be advantageously employed for diagnostic evaluation and/or treatment of subjects, in particular human subjects, because it is minimally invasive and may be safer (*e.g.*, less toxic) than other methods known in the art (*e.g.*, radioactive methods). In general, the inventive methods will be more readily accepted because they avoid radioactivity or toxic levels of chemicals or other agents-Subject according to the present invention can be any animal subject, and are preferably mammalian subjects (*e.g.*, humans, canines, felines, bovines, caprines, ovines, equines, rodents, porcines, and/or lagomorphs), and more preferably are human subjects.

The present invention is described with reference to flowchart illustrations and/or block diagrams of methods.

It will be understood that each block of the flowchart illustrations and/or block diagrams, and combinations of blocks in the flowchart illustrations and/or block diagrams, can be implemented by computer program instructions- These computer program instructions may be provided to a processor of a general purpose computer, special purpose computer, embedded processor or other programmable data processing apparatus to produce a machine, such that the instructions, which execute via the processor of the computer or other programmable data processing apparatus, create means for implementing the functions specified in the flowchart and/or block diagram block or blocks.

These computer program instructions may also be stored in a computer-readable memory that can direct a computer or other programmable data processing apparatus to function in a particular manner, such that the instructions stored in the computer-readable memory produce an article of manufacture including instruction means which implement the function specified in the flowchart and/or block diagram block or blocks.

The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions specified in the flowchart and/or block diagram block or blocks.

The present invention is a use of $^{129}$Xe in the manufacture of a medicament. The methods, systems and computer program products are examples useful for understanding the present invention.

The computer-usable or computer-readable medium may be, for example but not limited to, an electronic, magnetic, optical, electromagnetic, infrared, or semiconductor system, apparatus, device, or propagation medium. More specific examples (a non-exhaustive list) of the computer-readable medium would include the following: an electrical connection having one or more wires, a portable computer diskette, a random access memory (RAM), a read-only memory (ROM), an erasable programmable read-only memory (EPROM or Flash memory), an optical fiber, and a portable compact disc read-only memory (CD-ROM). Note that the computer-usable or computer-readable medium could even be paper or another suitable medium upon which the program is printed, as the program can be electronically captured, via, for instance, optical scanning of the paper or other medium, then compiled, interpreted or otherwise processed in a suitable manner if necessary, and then stored in a computer memory.

Computer program code for carrying out operations may be written in an object oriented programming language such as Java7, Smalltalk or C++. However, the computer program code for carrying out operations may also be written in conventional procedural programming languages, such as the "C" programming language or even assembly language. The program code may execute entirely on the user's computer, partly on the user's computer, as a stand-alone software package, partly on the user's computer and partly on a remote computer or entirely on the remote computer. In the latter scenario, the remote computer may be connected to the user's computer through a local area network (LAN) or a wide area network (WAN), or the connection may be made to an external computer (for example, through the Internet using an Internet Service Provider).

The flowcharts and block diagrams illustrate methods to obtain dynamic NMR signal data and analyze and evaluate the data to assess respiratory or cardiopulmonary function or disorders according to embodiments of the present invention. In this regard, each block in the flow charts or block diagrams represents a module, segment, or portion of code, which comprises one or more executable instructions for implementing the specified logical function(s). It should also be noted that in some alternative implementations, the functions noted in the blocks may occur out of the order noted in the figures. For example, two blocks shown in succession may in fact be executed substantially concurrently or the blocks may sometimes be executed in the reverse order, depending upon the functionality involved.

The invention is defined by the following claims.

**Claims**

1.  Use of $^{129}$Xe in the manufacture of a medicament for use in an *in vivo* method for evaluating whether a subject has a respiratory disorder, or a cardiopulmonary disorder such as chronic heart failure, comprising:

    delivering polarized $^{129}$Xe *in vivo* to a subject such that the polarized $^{129}$Xe travels across the alveolar-capillary membrane to be taken up in the blood across the membrane, the polarized $^{129}$Xe in the blood having a corresponding polarized $^{129}$Xe NMR chemical shift signal frequency; **characterised by**
    destroying the polarization of the polarized $^{129}$Xe in the blood and the membrane a plurality of times to define t=0 each time, wherein t=0 is the time when the polarization in the blood is destroyed;
    after each t=0, collecting data by transmitting an excitation pulse either later or earlier in time than the previous excitation pulse, such that a series of incrementally- or decrementally-spaced excitation pulses are transmitted to generate a response curve which maps the polarized $^{129}$Xe behaviour *in vivo* with at least millisecond resolution; and,
    evaluating the dynamic data to assess whether the subject has a respiratory or cardiopulmonary disorder such as chronic heart failure.

2.  The use according to Claim 1, wherein said method further comprises:

    calculating the time constant associated with the time it takes the polarized gas to travel across the membrane and then enter the blood after any one of said destroying steps; and,

determining the thickness of the membrane based on data provided by said obtaining and calculating steps.

3. The use according to Claim 2, wherein the step of collecting of said method comprises obtaining a plurality of signal data points over a time which is greater than: about twice the time constant.

4. The use according to either of Claims 2 or 3, wherein the collecting step of said method is carried out when the subject is at rest and then repeated while the subject is exposed to actual or simulated exercise, and wherein said method further comprises comparing the time constants associated therewith to thereby assess the function of the alveolar-capillary membrane.

5. The use according to any of Claims 2 to 4, wherein the step of determining of said method is used to measure membranes having a thickness in the range of about 1 micron to about 100 microns.

6. The use according to any of Claims 1 to 5, wherein the collecting step of said method is performed after a therapeutic agent is administered to the subject to evaluate the efficacy in treating the disorder or to evaluate its impact on the thickness of the alveolar-capillary membrane.

7. The use according to Claim 6, wherein the collecting step of said method is carried out both before and after the administration of the therapeutic to the subject.

8. The use according to Claim 1, wherein the evaluating step of said method comprises evaluating at least one of thickness of the alveolar-capillary membrane, perfusion in the pulmonary blood, ventilated blood oxygen saturation level, shunt, and ejection fraction, based on said collecting step.

**Patentansprüche**

1. Verwendung von $^{129}$Xe bei der Herstellung eines Medikaments zur Verwendung in einem *in vivo* Verfahren zwecks Evaluierens, ob ein Proband eine Atemwegserkrankung oder eine kardiopulmonale Erkrankung, wie zum Beispiel chronische Herzinsuffizienz, aufweist, umfassend:

Verabreichen von polarisiertem $^{129}$Xe *in vivo* an einen Probanden solchermaßen, dass sich das polarisierte $^{129}$Xe durch die alveolokapilläre Membran bewegt, um im Blut jenseits der Membran aufgenommen zu werden, wobei das polarisierte $^{129}$Xe im Blut eine entsprechende Signalfrequenz chemischer NMR-Verschiebung von polarisiertem $^{129}$Xe aufweist; **gekennzeichnet durch**
mehrmaliges Zerstören der Polarisation des polarisierten $^{129}$Xe in dem Blut und der Membran, um jedes Mal t = 0 festzulegen, wobei t = 0 der Zeitpunkt ist, zu dem die Polarisation im Blut zerstört ist;
nach jedem t = 0, Sammeln von Daten **durch** Übertragen eines Anregungsimpulses zeitlich entweder später oder früher als der vorherige Anregungsimpuls, so dass eine Serie zunehmend oder abnehmend beabstandeter Anregungsimpulse übertragen wird, um eine Reaktionskurve zu erzeugen, die das Verhalten des polarisierten $^{129}$Xe *in vivo* mit zumindest Millisekundenauflösung aufzeichnet; und
Evaluieren der dynamischen Daten zwecks Beurteilung, ob der Proband eine Atemwegserkrankung oder eine kardiopulmonale Erkrankung, wie zum Beispiel chronische Herzinsuffizienz, aufweist.

2. Verwendung nach Anspruch 1, wobei das Verfahren weiterhin umfasst:

Berechnen der Zeitkonstante, die mit der Zeit in Zusammenhang steht, die das polarisierte Gas benötigt, um sich durch die Membran zu bewegen und dann ins Blut zu dringen nach einem beliebigen der Zerstörungsschritte; und
Feststellen der Dicke der Membran auf Grundlage von Daten, die durch die Beschaffungs- und Berechnungsschritte bereitgestellt werden.

3. Verwendung nach Anspruch 2, wobei der Sammelschritt des Verfahrens das Erhalten einer Mehrzahl von Signaldatenpunkten über eine Zeit umfasst, die länger ist als etwa das Doppelte der Zeitkonstante.

4. Verwendung nach Anspruch 2 oder 3, wobei der Sammelschritt des Verfahrens durchgeführt wird, wenn der Proband ruht und dann wiederholt wird, während der Proband tatsächlicher oder simulierter Belastung ausgesetzt ist, und wobei das Verfahren weiterhin das Vergleichen der damit in Zusammenhang stehenden Zeitkonstanten umfasst, um **dadurch** die Funktion der alveolokapillären Membran zu beurteilen.

5. Verwendung nach einem der Ansprüche 2 bis 4, wobei der Feststellungsschritt des Verfahrens eingesetzt wird zum Messen von Membranen, die eine Dicke im Bereich von etwa 1 Mikron bis etwa 100 Mikron aufweisen.

6. Verwendung nach einem der Ansprüche 1 bis 5, wobei der Sammelschritt des Verfahrens durchgeführt wird, nachdem ein therapeutisches Agens dem Probanden verabreicht worden ist, um die Wirksamkeit beim Behandeln der Erkrankung zu evaluieren oder um seinen Einfluss auf die Dicke der alveolokapillären Membran zu evaluieren.

7. Verwendung nach Anspruch 6, wobei der Sammelschritt des Verfahrens sowohl vor als auch nach der Verabreichung des Therapeutikums an den Probanden durchgeführt wird.

8. Verwendung nach Anspruch 1, wobei der Evaluationsschritt des Verfahrens das Evaluieren zumindest einer Sache von Dicke der alveolokapillären Membran, Perfusion im pulmonalen Blut, Sauerstoffsättigungsgrad ventilierten Bluts, Shunt und Ausstoßfraktion umfasst, und zwar auf Grundlage des Sammelschritts.

**Revendications**

1. Utilisation de $^{129}$Xe dans la fabrication d'un médicament destiné à être utilisé dans un procédé d'évaluation in vivo du fait qu'un sujet présente un désordre respiratoire, ou un désordre cardio-pulmonaire tel qu'une défaillance cardiaque chronique, comprenant :

l'administration de $^{129}$Xe polarisé in vivo à un sujet de telle sorte que le $^{129}$Xe polarisé se déplace à travers la membrane alvéolaire-capillaire de manière à être assimilé dans le sang à travers la membrane, le $^{129}$Xe polarisé dans le sang présentant une fréquence de signal de décalage chimique de RMN du $^{129}$Xe polarisé correspondante ; **caractérisée par**
la destruction de la polarisation du $^{129}$Xe polarisé dans le sang et la membrane une pluralité de fois afin de définir t=0 chaque fois, dans laquelle t=0 représente l'instant auquel la polarisation dans le sang est détruite ; après chaque t=0, la collecte des données en transmettant une impulsion d'excitation soit plus tard ou plus tôt dans le temps que l'impulsion d'excitation précédente, telle qu'une série d'impulsions d'excitation espacées de manière incrémentale ou decrémentale est transmise afin de produire une courbe de réponse qui cartographie le comportement du $^{129}$Xe polarisé in vivo avec au moins une résolution en milliseconde ; et,
l'évaluation des données dynamiques afin de déterminer si le sujet présente un désordre respiratoire ou cardio-pulmonaire tel qu'une défaillance cardiaque chronique.

2. Utilisation selon la revendication 1, dans laquelle ledit procédé comprend, en outre :

le calcul de la constante de temps associée au temps pris par le gaz polarisé afin de traverser la membrane puis d'entrer dans le sang après l'une quelconque desdites étapes de destruction ; et,
la détermination de l'épaisseur de la membrane sur la base de données produites par lesdites étapes d'obtention et de calcul.

3. Utilisation selon la revendication 2, dans laquelle l'étape de collecte dudit procédé comprend l'obtention d'une pluralité de points de données de signal sur une période qui est supérieure au double environ de la constante de temps.

4. Utilisation selon l'une ou l'autre des revendications 2 ou 3, dans laquelle l'étape de collecte dudit procédé est mise en oeuvre lorsque le sujet est au repos puis elle est répétée alors que le sujet est soumis à un exercice réel ou simulé, et dans laquelle ledit procédé comprend, en outre, la comparaison des constantes de temps associées à cette dernière afin de déterminer ainsi la fonction de la membrane alvéolo-capillaire.

5. Utilisation selon l'une quelconque des revendications 2 à 4, dans laquelle l'étape de détermination dudit procédé est utilisée afin de mesurer des membranes présentant une épaisseur dans la plage de 1 micron environ à 100 microns environ.

6. Utilisation selon l'une quelconque des revendications 1 à 5, dans laquelle l'étape de collecte dudit procédé est exécutée après qu'un agent thérapeutique à été administré au sujet de manière à évaluer l'efficacité pour le traitement du désordre ou à évaluer son impact sur l'épaisseur de la membrane alvéolo-capillaire.

7. Utilisation selon la revendication 6, dans laquelle l'étape de collecte dudit procédé est mise en oeuvre à la fois avant et après l'administration de l'agent thérapeutique au sujet.

8. Utilisation selon la revendication 1, dans laquelle l'étape d'évaluation dudit procédé comprend l'évaluation d'au moins l'un de l'épaisseur de la membrane alvéolo-capillaire, de la perfusion dans le sang pulmonaire, du niveau de saturation en oxygène du sang ventilé, du shunt, et de la fraction d'éjection, sur la base de ladite étape de collecte.

FIG.1A

FIG.1B

FIG.2A

FIG.2B

FIG.2C

EP 1 430 322 B1

FIG.3

EP 1 430 322 B1

HIGH—RES VENTILATION IMAGE

FIG. 4A

XENON UPTAKE VS TIME CURVES

FIG. 4B

EP 1 430 322 B1

ADMINISTER POLARIZED 129Xe
TO A PATIENT IN VIVO.
200

DESTROY THE POLARIZATION OF
THE 129Xe IN THE BODY
LOCALLY PRIOR TO OBTAINING THE
DYNAMIC DATA.
222

OBTAIN AT LEAST ONE DYNAMIC DATA SET
OF AN NMR SPECTROGRAPHIC SIGNAL OF
THE POLARIZED 129Xe IN THE BODY OF THE
PATIENT AT AT LEAST ONE SELECTED
CHEMICAL SHIFT FREQUENCY OVER TIME.
220

EVALUATE THE DYNAMIC DATA
TO DETERMINE WHETHER THE
SUBJECT HAS A
CARDIOPULMONARY DISORDER
SUCH AS CHRONIC HEART
FAILURE.
230

OBTAIN TWO DYNAMIC DATA SETS, ONE
EACH AT TWO DIFFERENT RESPECTIVE
FREQUENCIES, ONE CORRESPONDING TO
POLARIZED 129Xe IN LUNG TISSUE AND
THE OTHER CORRESPONDING TO
POLARIZED 129Xe IN PULMONARY
BLOOD.
235

EVALUATE AT LEAST ONE OF THE
TIME CONSTANT, TRANSIT TIME,
LINE SHAPE, OR SLOPE(S), AREA
UNDER THE CURVE, AND
SELECTED AMPLITUDES, OF THE
SIGNAL AT AT LEAST ONE
SELECTED CHEMICAL SHIFT
FREQUENCY CORRESPONDING TO
THE DYNAMIC DATA.
233

OBTAIN THE TISSUE AND BLOOD DYNAMIC
DATA SETS TWICE, ONCE WHEN THE
PATIENT IS SUBSTANTIALLY AT REST AND
ONE WHEN THE PATIENT IS UNDER STRESS,
AND COMPARING THE DATA SETS TO
DETERMINE IF THE SUBJECT HAS CHRONIC
HEART FAILURE.
238

ADMINISTER A THERAPEUTIC
AGENT AND REPEAT BLOCK 235.
240

FIG.5

20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20010000727 A
- US 5642625 A, Cates
- US 5809801 A, Cates
- US 6079213 A, Driehuys
- US 5545396 A, Albert
- US 271476 A
- US 5833947 A
- US 5522390 A
- US 5509412 A
- US 5494655 A
- US 5352979 A
- US 5190744 A
- US 804369 A
- US 09271476 A

### Non-patent literature cited in the description

- The Task Force on Heart Failure of the European Society of cardiology. Guidelines for the Diagnosis of Heart Failure. *Eur. Heart Jnl.,* 1995, vol. 16, 741-751
- **Tan et al.** Heart Failure in Elderly Patients: Focus on diastolic Dysfunction, Heart Failure: Scientific Principles and Clinical Practice. 1997
- **Wagshul et al.** *Magnetic Resonance in Medicine,* 1996, vol. 36 (2), 183-191
- **Swanson et al.** *Magnetic Resonance in Medicine,* 1999, vol. 42, 1137-45
- **Ruppert et al.** NMR of Hyperpolarized Xe in the Canine Chest: Spectral Dynamics During a Breath-Hold. *13 NMR in Biomediciene,* 2000, 623-641
- **Wolber et al.** Measuring Diffusion of Xenon in solution with hyperpolarized 129Xe NMR. *296 Chemical Physics Letters,* 06 November 1998, 391-396
- **Hou et al.** Optimization of Fast Acquisition Methods for Whole-Brain Relative Cerebral Blood Volume (rCBV) Mapping with Susceptibility Contrast Agents. *9 J. Magnetic Resonance Imaging 233,* 1999
- **Simonsen et al.** CBF and CBV Measurements by USPIO Bolus Tracking: Reproducibility and Comparison with Gd-Based Values. *9 J. Magnetic Resonance Imaging 342,* 1999
- **Mugler III et al.** MR Imaging and Spectroscopy Using Hyperpolarized Xe gas: Preliminary Human Results. *37 Magnetic Resonance in Medicine,* 1997, 809-815
- **Belliveau et al.** Functional Cerebral Imaging by Susceptibility-Contrast NMR. *14 Magnetic Resonance in Medicine 14 538,* 1990
- **Detre et al.** Measurement of Regional Cerebral Blood Flow in Cat Brain Using Intracarotid H20 and H NMR Imaging. *14 Magnetic Resonance in Medicine 389,* 1990
- **Frank et al.** Dynamic Dysprosium-DTPA-BMA Enhanced MRI of the Occipital Cortex; Functional Imaging in Visually Impaired Monkeys by PET and MRI (Abstract). *Ninth Annual Scientific Meeting and Exhibition of the Society of Magnetic Resonance In Medicine,* 18 August 1990
- **Rosen et al.** Perfusion Imaging by Nuclear Magnetic Resonance. *5 Magnetic Resonance Quarterly 263,* 1989
- **Lassen.** Cerebral Transit of an Intravascular Tracer may Allow Measurement of regional Blood Volume but flot Regional Blood Flow. *J. Cores. Blood Flow and Metab.,* 1984, vol. 4, 633